# FASCICULE DE BREVET EUROPEEN

(11) **EP 2 436 347 B1**
(45) Date de publication et mention de la délivrance du brevet: **10.01.2018**
(21) Numéro de dépôt: 11171380.6
(22) Date de dépôt: 24.06.2011
(51) Int. Cl.: A61F 11/08, A61F 11/10

(54) **Pièce tampon à insérer dans un bouchon d'oreille permettant l'écrêtage de pics acoustiques forts et brefs par déphasage d'ondes**
Stopfenteil, der in einen Gehörschutzstöpsel eingeschoben wird und das Kappen von lauten und kurzen Geräuschspitzen durch Phasenverschiebung der Schallwellen ermöglicht
Plug insert to be inserted in an earplug for clipping intense, brief acoustic peaks by wave phase displacement

(30) Priorité: 30.09.2010 FR 1003890
(43) Date de publication de la demande: 04.04.2012
(73) Titulaire: Roussel, Pascal, 76160 Saint Martin du Vivier (FR)
(72) Inventeur: Roussel, Pascal, 76160 Saint Martin du Vivier (FR)
(74) Mandataire: Novagraaf Technologies

(56) Documents cités:
- DE-U1- 8 536 433
- DE-U1- 20 200 638
- FR-A- 386 276
- GB-A- 191 404 821
- US-A1- 2003 159 878

## Description

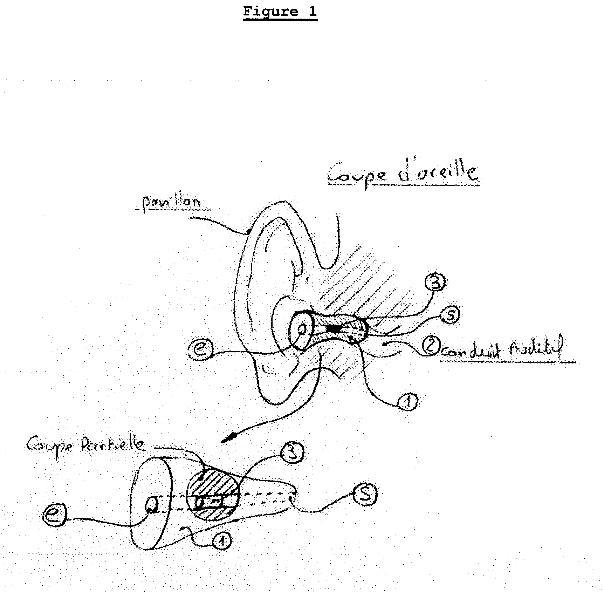

La présente invention concerne un dispositif à insérer dans un bouchon d'oreille (1.Fig1) de type mousse, acrylique ou silicone épousant parfaitement les formes du conduit auditif (2.Fig1) et formant une parfaite étanchéité au son. Le bouchon d'oreille sera ensuite percer de part et d'autre (e&s.Fig1) afin d'y insérer la présente invention (3.Fig1) permettant l'écrêtement de pics acoustiques impulsionnels.

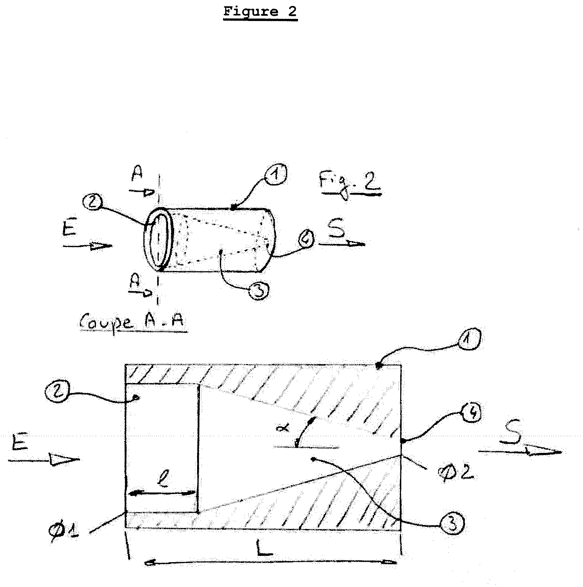

On connaît aujourd'hui différents systèmes de protection antibruit standard ou sur mesure permettant la protection aux nuisances sonores d'opérateurs ou salariés exposés. Il existe aussi des protections antibruit avec un asservissement électronique permettant de compresser le signal dès qu'il devient très fort, de telle protection de part leur coût sont utilisées dans le maniement d'arme à feu comme à la chasse ou au ball-trap. Il existe aussi des protections antibruit spécifiquement étudiées pour la protection de l'audition et des communications pour les militaires lors d'explosions pouvant atteindre plus de 170 dB SPL. Pour les personnels exposés aux bruits de l'industrie, les mesures acoustiques habituellement faites permettant de proposer l'antibruit adéquat répondent à la règlementation en tant que protection à la dose de bruit journalière notée Leq(x) ; les réglementations Européennes visant à protéger les opérateurs exposés aux bruits prennent aussi maintenant en compte les nuisances sonores de type impulsionnels notées Lpcmax, il n'existe pas actuellement de dispositif traitant spécifiquement ce problème et apportant aux salariés et opérateurs exposés à des bruits forts et impulsionnels des protecteurs antibruit adaptés à ces nuisances, les fréquences et les intensité étant spécifiques à de tels métiers. Les bruits en question sont généralement provoqués par le travail du métal, l'échappement d'air comprimé, les impacts de burin ou marteau perforateur dans le BTP, etc ... les valeurs réglementaires actuelles sont 135 dB(C) pour une première action de prévention jusqu'à 140 dB(C) valeur maximum autorisée à l'oreille. Des bouchons d'oreille sont connus des documents DE-U-8536433, US-A-2003/159878, DE-U-20200638. L'invention consiste en une pièce unique (1.Fig2), usinée en métal, alliage, cuivre, aluminium ou injectée en matière plastique de type ABS de forme extérieure cylindrique et de forme intérieure spécifique. Cette pièce n'ayant pas d'influence particulièrement importante pour les sons de type continus mais ayant un effet d'écrêtement pour les pics impulsionnel de type « coup de marteau » sur une pièce métallique par exemple. La forme spécifique intérieure de la pièce est composée d'une entrée cylindrique (2.Fig2) suivie d'un cône (3.Fig2) dit « cône de compression acoustique » et d'une sortie (4.Fig2) à la pointe dudit cône. La pièce a un sens de fonctionnement (E>S.Fig2), les pics impulsionnels du bruit entrant dans la partie cylindrique (2.Fig2) la plus large, puis sortant écrêtés par la pointe du cône (4.Fig2) vers le tympan de l'oreille. Le système d'écrêtement fonctionne à partir de pics de moins de 0,5 ms avec plus de 90 dB SPL d'intensité ; les valeurs maximales d'écrêtement sont obtenues pour des bruits impulsionnels de trains d'ondes générés sur la base de 3000 à 6000 Hz en fréquence fondamentales comme des bruits métalliques. La forme de cône dit «cône de compression acoustique» (3.Fig2) conditionne l'efficacité dudit dispositif, une adaptation au type de bruit devant être écrêté sera faite par variation de la longueur totale (L.Fig2) extérieure du cylindre de 10 à 5 mm et de 3 à 5 mm de diamètre, par l'angle du cône (Alpha.Fig2) obtenu par la modification de la longueur de l'entrée cylindrique (l.Fig2) de 1 à 3 mm, de son diamètre intérieur (Diam1.fig2) de 1 à 3 mm et du diamètre de la pointe du cône (4.Fig2) de 0,1 à 0,8 mm.
Dans ces conditions un pic impulsionnel d'entrée de type métallique de 140 dB(C) de LpcMax sera écrêté de 5 à 10 %. Plus précisément, le diamètre de la forme extérieure cylindrique est compris entre 3 et 5 mm.
Le cylindre d'entrée (2.Fig2) de la forme intérieure présente, quand à lui, un diamètre intérieur (diam1.Fig2) compris entre 1 à 3 mm et une longueur (l.Fig2) comprise entre 1 à 3 mm.

## Revendications

1. Dispositif destiné à écrêter les pics acoustiques impulsionnels et à être inséré dans le perçage d'un bouchon d'oreille étanche, ledit dispositif comprenant un système acoustique de forme extérieure cylindrique (1) de 5 à 10 mm de longueur (L) et de 3 à 5 mm de diamètre et d'un usinage intérieur constitué, dans le sens (E-S) de traitement de l'onde acoustique, d'une entrée cylindrique (2), d'un cône de compression acoustique (3) et d'une sortie (4) située à la pointe dudit cône.

2. Dispositif selon la revendication 1 **caractérisé en ce que** l'entrée cylindrique a une longueur (1) comprise entre 1 et 3 mm et un diamètre intérieur (Dl) compris entre 1 et 3 mm.

3. Dispositif selon l'une des revendications 1 et 2 **caractérisé en ce que** la pointe de sortie du cône de compression (3) a un diamètre (D2) compris entre 0,1 et 0,8 mm.

4. Dispositif selon l'une des revendications précédentes caractérisé en qu'il est réalisé en métal, alliage ou matière plastique.

5. Dispositif selon l'une des revendications précédentes caractérisé en qu'il consiste en une pièce unique.

6. Dispositif selon la revendication 4 **caractérisé en ce que** ladite pièce unique est usinée en métal, alliage, cuivre, aluminium ou injectée en matière plastique de type ABS.

7. Bouchon d'oreille étanche **caractérisé en ce qu'**il comporte un perçage dans lequel est inséré, un dispositif destiné à écrêter les pics acoustiques impulsionnels selon l'une des revendications précédentes.

## Patentansprüche

1. Vorrichtung, die dazu bestimmt ist, die impulsartigen Schallspitzen zu kappen und in die Bohrung eines dichten Gehörschutzstöpsels eingeschoben zu werden, wobei die Vorrichtung ein Schallsystem mit zylindrischer Außenform (1) von 5 bis 10 mm Länge (L) und 3 bis 5 mm Durchmesser und mit einer Innenausarbeitung umfasst, die sich in der Richtung (ES) der Verarbeitung der Schallwelle aus einem zylindrischen Eingang (2), einem Schallkompressionskegel (3) und einem an der Spitze des Kegels liegenden Ausgang (4) zusammensetzt.

2. Vorrichtung nach Anspruch 1, **dadurch gekennzeichnet, dass** der zylindrische Eingang eine Länge (1) im Bereich zwischen 1 und 3 mm und einen Innendurchmesser (D1) im Bereich zwischen 1 und 3 mm besitzt.

3. Vorrichtung nach einem der Ansprüche 1 und 2, **dadurch gekennzeichnet, dass** die Ausgangsspitze des Kompressionskegels (3) einen Durchmesser (D2) im Bereich zwischen 0,1 und 0,8 mm besitzt.

4. Vorrichtung nach einem der vorstehenden Ansprüche, **dadurch gekennzeichnet, dass** sie aus Metall, Legierung oder Kunststoff ausgeführt ist.

5. Vorrichtung nach einem der vorstehenden Ansprüche, **dadurch gekennzeichnet, dass** sie aus einem einzigen Stück besteht.

6. Vorrichtung nach Anspruch 4, **dadurch gekennzeichnet, dass** das einzige Stück aus Metall, Legierung, Kupfer, Aluminium ausgearbeitet oder aus Kunststoff vom Typ ABS spritzgegossen ist.

7. Dichter Gehörschutzstöpsel, **dadurch gekennzeichnet, dass** er eine Bohrung umfasst, in die eine Vorrichtung nach einem der vorstehenden Ansprüche eingeschoben ist, die dazu bestimmt ist, die impulsartigen Schallspitzen zu kappen.

## Claims

1. Device intended to clip pulsed acoustic peaks and to be inserted in the hole of a waterproof earplug, said device comprising an acoustic system having a cylindrical external shape (1) of 5 to 10 mm in length (L) and of 3 to 5 mm in diameter and an internal machining consisting, in the acoustic wave processing direction (E-S), of a cylindrical inlet (2), an acoustic compression cone (3) and an outlet (4) situated at the tip of said cone.

2. Device according to claim 1 **characterised in that** the cylindrical inlet has a length (1) between 1 and 3 mm and an internal diameter (D1) between 1 and 3 mm.

3. Device according to one of claims 1 and 2 **characterised in that** the outlet tip of the compression cone (3) has a diameter (D2) between 0.1 and 0.8 mm.

4. Device according to one of the preceding claims **characterised in that** it is made of metal, alloy or plastic.

5. Device according to one of the preceding claims **characterised in that** it consists of a single piece.

6. Device according to claim 4 **characterised in that** said single piece is machined from metal, alloy, copper, aluminium or injection-moulded from ABS type plastic.

7. Waterproof earplug **characterised in that** it comprises a hole wherein is inserted, a device for clipping pulsed acoustic peaks according to one of the preceding claims.
